# EUROPEAN PATENT APPLICATION

(11) **EP 1 256 632 A2**
(43) Date of publication of application: **13.11.2002**
(21) Application number: 02076698.6
(22) Date of filing: 02.05.2002
(51) Int. Cl.: C12Q 1/68

(54) **High throughput polymorphism screening**

(30) Priority: 07.05.2001 US 289606 P
(71) Applicant: SMITHKLINE BEECHAM CORPORATION, Philadelphia Pennsylvania 19103 (US)
(72) Inventor: Jones, Keith, Research Triangle Park, NC 27709 (US); Leuther, Kerstin K., Research Triangle Park, NC 27709 (US); Shapero, Michael H., Research Triangle Park, NC 27709 (US)
(74) Representative: Giddings, Peter John, Dr.

(57) **Abstract**

Methods are provided for determining the identity of a polymorphic nucleotide in a complex mixture of nucleic acids where one or more distinct polymorphisms can be present in the mixture, and multiple polymorphisms can be screened in parallel. Target nucleic acids are amplified using bridge amplification techniques. The detection and identification of the specific polymorphic residue(s) is based on readout methods that utilize the specificity of specific enzymes for complementary DNA sequences. These approaches result in a labeled nucleotide covalently attached to the amplicon, where the identity of the nucleotide is informative of the polymorphic sequence. In one aspect, the readout process uses primer extension protocols, where the specific base incorporated by DNA polymerase is determined by the sequence at the polymorphic site. In another aspect, the identity of a specific base hybridized and ligated to the amplicon is determined by the sequence at the polymorphic site. The polynucleotide to which the label has been attached can be detected *in situ,* i.e. bound to the solid substrate used for amplification; or can be released and detected.

SEQUENCE LISTING

<110> Keith W. Jones
      Kerstin K. Leuther
      Michael H. Shapero

<120> HIGH THROUGHPUT POLYMORPHISM SCREENING

<130> PK4581US2

<140> to be assigned
<141>

<150> 60/289,606
<151> 2001-05-07

<160> 23

<170> FastSEQ for Windows Version 4.0

<210> 1
<211> 47
<212> DNA
<213> homo sapien

<400> 1
ttttttgata tcgcagcaga gggactggag aagcatacac ttctgat                47

<210> 2
<211> 49
<212> DNA
<213> homo sapien

<400> 2
atcagaagtg tgtatgcttc tccagtccct ctgctgcgat atcaaaaaa             49

<210> 3
<211> 37
<212> DNA
<213> homo sapien

<400> 3
taggcctaca ccaggctcta cagcagcgac tttaact                          37

<210> 4
<211> 35
<212> DNA
<213> homo sapien

<400> 4
tcagctgggc acccttcaca ggaaaggttg gttcg                            35

<210> 5
<211> 42
<212> DNA
<213> homo sapien

<400> 5
ttttttaggc cttcagaggc tcctgcagca gccagagagc tc                     42

<210> 6
<211> 42
<212> DNA
<213> homo sapien

<400> 6
ttttttcagc tggaatacct gatttcacaa tcaagttaaa gg                    42

<210> 7
<211> 42
<212> DNA
<213> homo sapien

<400> 7
ttttttaggc ctttcaaccc ctctaccgca gcccacacag gt                    42

<210> 8
<211> 40
<212> DNA
<213> homo sapien

<400> 8
ttttttcagc tgcctcccac ctctccacct cccgagtagc                       40

<210> 9
<211> 42
<212> DNA
<213> homo sapien

<400> 9
ttttttcagc tgatggaaat cacacagtca caaaggagca ga                    42

<210> 10
<211> 43
<212> DNA
<213> homo sapien

<400> 10
ttttttaggc cttggactca cgaaggcgca gccggtgacc att                  43

<210> 11
<211> 42
<212> DNA
<213> homo sapien

<400> 11
ttttttaggc ctacaccagg ctctacagca gcgactttaa ct                    42

<210> 12
<211> 41
<212> DNA
<213> homo sapien

<400> 12
tttttcagct ggttggtggc attctgcggc tttttctcta g                    41

<210> 13
<211> 42
<212> DNA
<213> homo sapien

<400> 13
ttttttcagc tgagggccgc tccctcctca ttcctgtctt tc                42

<210> 14
<211> 43
<212> DNA
<213> homo sapien

<400> 14
ttttttaggc ctagccgggg ttggcccgca gccgcaggga gac               43

<210> 15
<211> 42
<212> DNA
<213> homo sapien

<400> 15
ttttttaggc ctactgcatg gtcccttgca gcagtggaca cc                42

<210> 16
<211> 40
<212> DNA
<213> homo sapien

<400> 16
ttttttcagc tgggcaaaca ccttctgaaa gtcggcgttg                   40

<210> 17
<211> 40
<212> DNA
<213> homo sapien

<400> 17
ttttttcagc tgataagcat caagtgtttg gaacagtgcc                   40

<210> 18
<211> 43
<212> DNA
<213> homo sapien

<400> 18
ttttttaggc ctagaggaag gagtggcgca gcgttcccta gtc               43

<210> 19
<211> 42
<212> DNA
<213> homo sapien

<400> 19
ttttttaggc ctctgtgccg ccttcatgca gctctttctc gg                42

<210> 20
<211> 40
<212> DNA
<213> homo sapien

<400> 20
ttttttcagc tggagggtcg ctagggccgc aggagggtta                   40

<210> 21

<211> 47
<212> DNA
<213> homo sapien

<400> 21
aaaaaactat agcgtcgtct ccctgacctc ttcgtatgtg aagacta 47

<210> 22
<211> 25
<212> DNA
<213> homo sapien

<400> 22
tttttgata tcgcagcaga gggac 25

<210> 23
<211> 29
<212> DNA
<213> homo sapien

<400> 23
aaaaaactat agcgtcgtct ccctgacct 29

## Description

### FIELD OF THE INVENTION

The invention relates to methods for determining the sequence of polymorphisms in a nucleic acid sample.

### BACKGROUND OF THE INVENTION

The ability to discern genetic variation among individuals is fundamental to achieving a better understanding of genetic predisposition to complex diseases. There are many types of DNA sequence variation between individuals, including deletions, insertions, short tandem repeats such as variable number tandem repeats (VNTR) and microsatellite di-and tri-nucleotide repeats, and the like. An emerging class of markers for genetic analysis are the single nucleotide polymorphism (SNP) and other simple polymorphisms, *e.g.* deletions, double nucleotide polymorphisms, *etc.* SNPs are generally biallelic systems, that is, there are two alleles in a population for any particular marker. This means that the information content per SNP marker is relatively low when compared to microsatellite markers, which may have upwards of 10 alleles, although their frequency in a population is very high. SNPs also tend to be very population-specific; a marker that is polymorphic in one population may not be polymorphic in another.

A major effort of the Human Genome Project along with completion of the human genome DNA sequence is the generation of high-density, evenly spaced SNP maps. Identification and mapping of novel SNPs will be followed by determination of allele frequencies in ethnically diverse populations for a subset of markers. These maps will provide the framework for powerful new association studies to identify genes in complex polygenic diseases, such as the link between APOE alleles and late onset familial Alzheimers disease (Corder, *et al.* (1993) *Science* 261:921-923). SNPs, found approximately every kilobase (see Wang *et al.* (1998) *Science* 280:1077-1082), offer the potential for generating very high density genetic maps, which will be extremely useful for developing haplotyping systems for genes or regions of interest, and because of the nature of SNPs, they may in fact be the polymorphisms associated with the disease phenotypes under study. The low mutation rate of SNPs also makes them excellent markers for studying complex genetic traits.

The field of pharmacogenomics relies heavily on the detection of SNPs. For example, single nucleotide polymorphisms in CYP2D6 can be used to predict individual drug metabolism rates. CYP2D6, which may be involved in metabolism of ∼25% of drugs including antiarrhythmics, antidepressants, beta-adrenergic blockers, and neuroleptics, can have enzymatic activity that spans a range from complete deficiency to ultrahigh levels depending on which of at least 16 different allelic variants are present. Screening individuals to identify poor metabolizers, who are at risk of adverse events at recommended therapeutic drug doses, can reduce the time and cost of clinical drug trials.

The wide-ranging applications of SNPs in pharmacogenetics and candidate and genome-wide disease susceptibility gene identification will necessitate further development of robust, flexible, cost-effective technology platforms for scoring genotypes in large numbers of samples. A variety of molecular genotyping schemes have been described over the years (reviewed in Landegren, *et al.* (1998) *Genome Res.* 8:769-776), many of which include bead-based and solid-phase approaches such as rolling circle amplification (Hatch, *et al.* (1999) *Genetic Analysis: Biomolecular Engineering* 15:35-40), flow cytometry and single base chain extension (Chen, *et al.* (2000) *Genome Res.* 10:549-557; lannone, *et al.* (2000) *Cytometry* 39:131-140), high-density oligonucleotide arrays (Wang, *et al.* (1998) *Science* 280:1077-1082; Fan, *et al.* (2000) *Genome Res.* 10:853-860), and fiber-optic gene arrays (Steemers, *et al.* (2000) *Nat. Biotechnol.* 18:91-94). While these methods may offer the capability of accurate genotyping, they all also rely on standard PCR amplification of target sequences as the initial front-end step in generating material. This inherent requirement effectively limits the extent to which these varied platforms can be modified for highly multiplexed genotyping, due to the technically demanding nature of standard solution-based multiplexed PCR.

The ability to convert hundreds of PCR primer-pairs into a single-tube multiplexed reaction producing specific, robust reaction products from a complex genomic DNA template would greatly ease the requirements for large-scale population-based SNP genotyping. As such, there is a need for the development of accurate high-throughput methods of SNP genotyping.

### SUMMARY OF THE INVENTION

Methods are provided for determining the identity of a polymorphic nucleotide in a complex mixture of nucleic acids where one or more distinct polymorphisms can be present in the mixture, and multiple polymorphisms can be screened in parallel. The subject methods employ solid-phase Bridge amplification techniques, utilizing a solid substrate with bound first and second locus-specific primers. Each single-stranded locus-specific primer has a free end, and an end bound to the substrate. The first primer comprises a region of sequence complementarity to one strand of its target nucleic acid, and the second primer comprises a region of complementarity with the opposite strand of the target nucleic acid. Target nucleic acids are amplified by the process of hybridization, extension and denaturation from the two primers. The double-stranded amplification product, or amplicon, is then contacted with a labeled nucleotide or nucleotides under conditions that permit specific binding to the amplicon. This readout of the polymorphic sequence may utilize either a primer extension protocol, or a ligation protocol. For either readout protocol, the amplicon after sequence specific labeling can be retained on the solid support, or released for detection.

The primer extension protocol can utilize an exogenous primer, or through manipulation of overhanging ends left from endonuclease cleavage can provide a primer endogenous to the amplicon. In one embodiment, the double-stranded amplification product is denatured prior to attachment of the labeled probe, and an exogenous extension primer complementary to a site immediately adjacent to the targeted polymorphic site is hybridized to the denatured amplicon. The extension primer initiates DNA synthesis across the polymorphic site in the presence of labeled dideoxynucleotides. Detection of the specific dideoxy base(s) incorporated in the synthesis reaction indicates the identity of the polymorphic nucleotide(s) in the target nucleic acid.

In another primer extension protocol, an endonuclease recognition site is provided for, so that the first nucleotide on the single-stranded overhang of the cleavage product is the polymorphic site (i.e., a one nucleotide extension of the recessed strand will base pair with the polymorphic site). The recessed strand can be used to initiate DNA synthesis across the polymorphic site in the presence of labeled dideoxynucleotides.

In another embodiment, using the ligation readout, a recognition site for an endonuclease that cleaves at a site distant from the recognition site and leaves staggered ends after cleavage is provided for, near the free end of one locus-specific primer. The endonuclease is selected to provide for cleavage at a position adjacent to the polymorphic site, such that the polymorphic site is on the single-stranded overhang of the cleavage product. The overhang can be hybridized to labeled probes comprising all possible combinations of the overhang nucleotide sequence, and ligated. Preferably, a label is attached to the probe so that the nucleotide sequence at the polymorphic site can be determined.

An advantage of the subject methods is that each locus to be tested does not have to undergo an initial separate amplification or a solution-based multiplexed amplification. Another advantage of the subject methods is that numerous polymorphic sites can be analyzed simultaneously in a single reaction chamber.

These and other objects, advantages, and features of the invention will become apparent to those persons skilled in the art upon reading the details of the method as more fully described below.

### BRIEF DESCRIPTION OF THE DRAWINGS

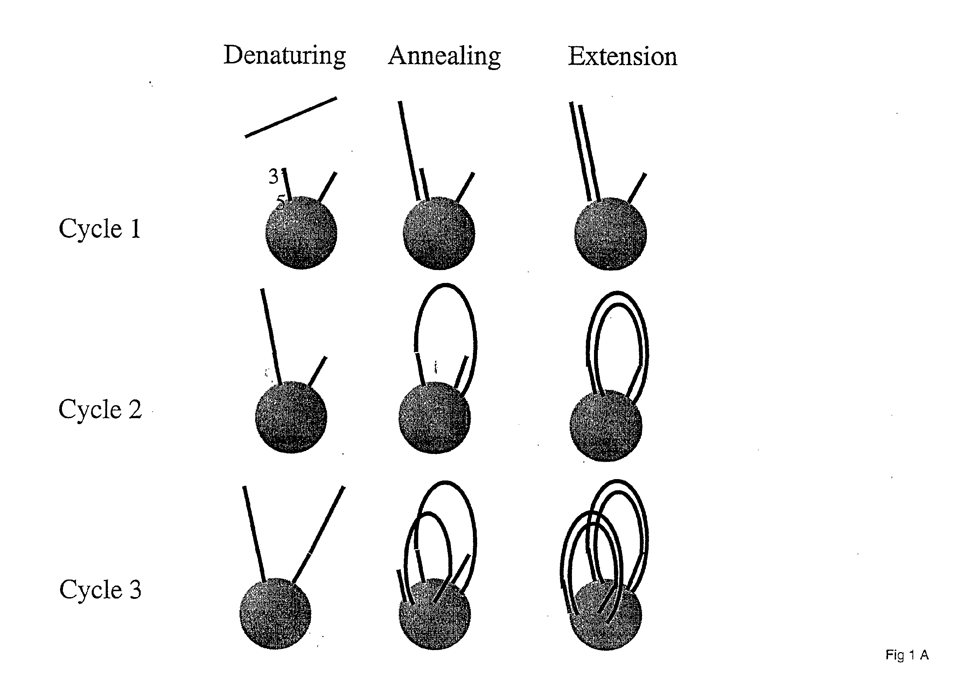

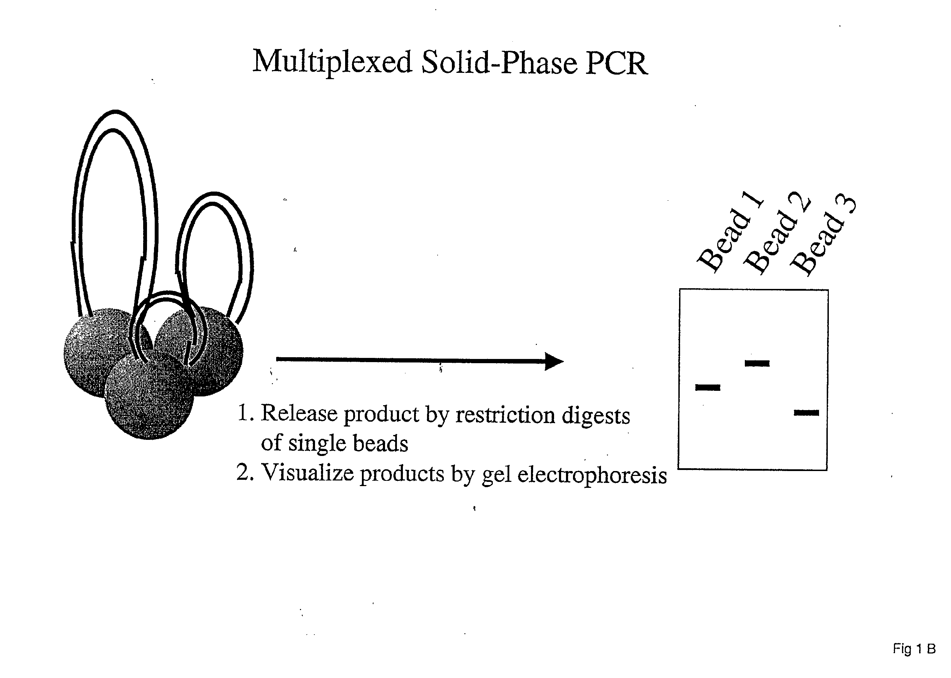

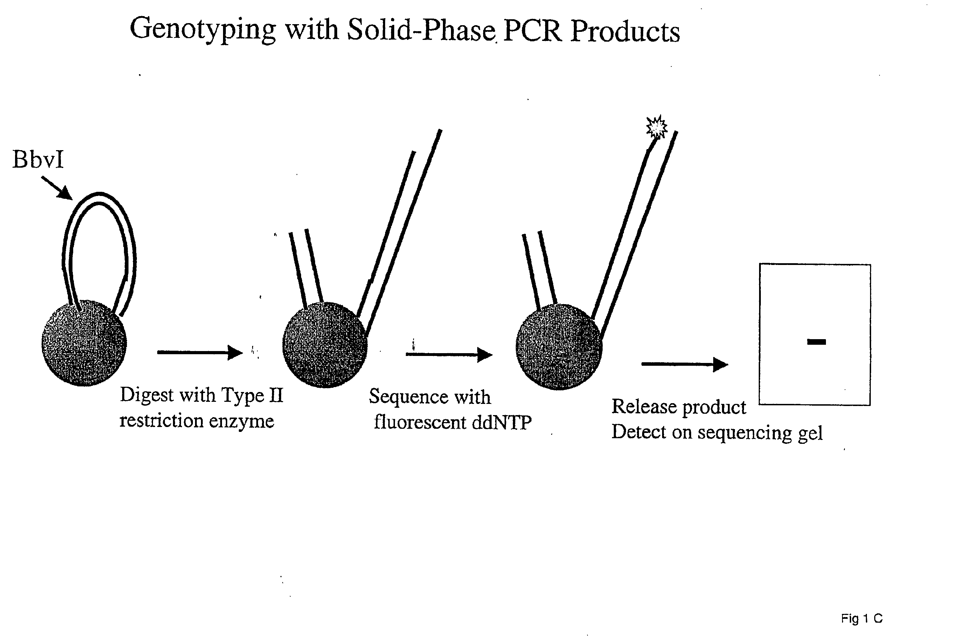

Figure 1 is a schematic depicting the following methods: (A) solid-phase amplification, (B) multiplexed bridge amplification, and (C) genotyping with solid-phase amplification products.
Figure 2 depicts the nucleic acid products of multiplex solid-phase PCR with human genomic DNA.
Figure 3A is a schematic of the sequence of an artificial duplex on a bead surface containing Type IIS restriction enzyme sites and the expected sequence following Bbvl digestion. Figures 3B-E are fluorescent images showing single color SNP minisequencing.
Figure 4 is a photographic image of released solid-phase PCR/minisequencing products separated on an acrylamide gel.

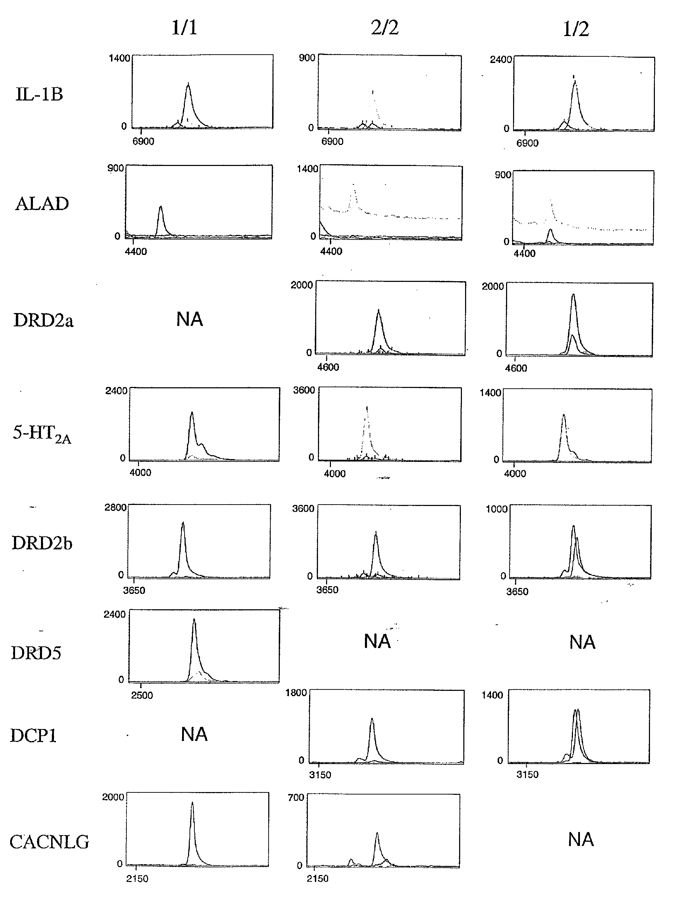

Figure 5. Electropheragrams for representative genotypes of each locus are shown. The Y-axis denotes relative fluorescent intensity, and the X-axis denotes scan number. The X-axis is not fully labeled,, but is shown left to right in the direction of increasing time to detection (increasing fragment length). The numerical genotype designations are indicated in Table 1 notes. The *Bbv*I recognition site was placed into the forward primer in the ALAD, 5-HT₂ₐ, IL1B, DRD5 and CACNLG amplicons. This resulted in fluorescent labeling of the noncoding strand such that blue electropheragram tracings indicated a C allele, red tracings indicated an A allele, green tracings indicated a T allele and black (yellow) tracings indicated a G allele. The *Bbv*I recognition site was placed into the reverse primer for the DRD2a, DRD2v, and DCP1 amplicons. This resulted in fluorescent labeling of the coding strand such that blue electropheragram tracings indicated a G allele, red tracings indicated a T allele, green tracings indicated an A allele and black (yellow) tracings indicated a C allele.

### DETAILED DESCRIPTION OF PREFERRED EMBODIMENTS

Methods are provided for determining the identity of polymorphic nucleotide(s) in a complex mixture of nucleic acids, using solid-phase bridge amplification techniques. Different approaches are used for detection and identification of the specific polymorphic residue(s). Both readout approaches exploit the specificity of specific enzymes for complementary DNA sequences, and both approaches result in a nucleotide, which may be referred to as a detecting nucleotide, covalently attached to the amplicon, where the identity of the nucleotide is informative of the polymorphic sequence. Conveniently, the detecting nucleotide comprises a label that is indicative of the base identity. In one aspect, the readout process uses primer extension protocols, where the specific base incorporated by DNA polymerase is determined by the sequence at the polymorphic site. In another aspect, the identity of a specific base hybridized and ligated to the amplicon is determined by the sequence at the polymorphic site. The polynucleotide to which the label has been attached can be detected *in situ*, i.e. bound to the solid substrate used for amplification; or can be released and detected.

In the primer extension methods, the double-stranded amplification product may be denatured and hybridized with a primer immediately adjacent to the targeted polymorphic site, which primer is used to initiate DNA synthesis across the polymorphic site in the presence of labeled dideoxynucleotides. Detection of the incorporated dideoxy base(s) allows identification of the polymorphic nucleotide(s). In another embodiment, the first nucleotide on the single-stranded overhang of a cleavage product is the polymorphic site (*i.e.*, a one nucleotide extension of the recessed strand will base pair with the polymorphic site). The recessed strand is used to initiate DNA synthesis across the polymorphic site in the presence of labeled dideoxynucleotides.

Using ligation methods, one locus-specific primer is designed to include a recognition site for an endonuclease that cleaves at a site distant from the recognition site and adjacent to the targeted polymorphic site. After amplification and cleavage with the endonuclease, the polymorphic site is on a single-stranded overhang, which can be hybridized and ligated to labeled probes for detection.

Within a species, there are genetic sites that are polymorphic, i.e. within a population, more than one nucleotide (G, A, T, C) is found at a specific position. Polymorphisms may be substitution, addition or deletion of one or more nucleotides at a particular site. Frequently the detected variation will be a point mutation, or single nucleotide polymorphism. However, small deletions, additions, and multiple nucleotide variations are also detected.

Many polymorphisms have been identified and some have been linked to known diseases. For example, sickle cell anemia, cystic fibrosis, and diabetes have all been linked to genomic polymorphisms. However, many polymorphisms have yet to be analyzed for association with disease. Knowledge of genetic variation in an individual is important not only for diagnosis of genetic predisposition to many diseases, but also for genetically controlled differences in metabolism and response to therapeutic agents. Variation can also be used to determine which genes contribute to multigenic or quantitative traits such as increased susceptibility to diseases or for understanding why some strains of a microbe are exceptionally virulent. For example, linkage analysis of polymorphism genotypes in a diseased and control population can be used to narrow down the area of search on a chromosome for a disease-associated gene (Riley, *et al.* (2000) *Pharmacogenetics* 1:39-47). Furthermore, genetic variation can be employed for diagnostics, identification purposes, both in microbiology and in forensics, for studies of recombination, and in population genetics. The methods of the present invention allow simultaneous and efficient screening of a complex mixture of nucleic acids for many known polymorphisms in a single reaction chamber. Also provided are kits for use in screening a nucleic acid sample for known polymorphisms.

### DEFINITIONS

Before the present methods are described, it is to be understood that this invention is not limited to particular methods described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are herein incorporated by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a locus-specific primer" includes a plurality of such locus-specific primers and reference to "a polymorphic site" includes reference to one or more polymorphic sites and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

*Nucleic acid.* A polymer of nucleotides, *e.g.* deoxyribonucleotides, ribonucleotides, or analogs thereof, including compounds produced synthetically that can hybridize with naturally occurring nucleic acids in a sequence-specific manner. The term includes, but is not limited to, single-, double-, or multi-stranded DNA or RNA, genomic DNA, cDNA, DNA-RNA hybrids, polymers comprising purine and pyrimidine bases or other natural, chemically or biochemically modified, non-natural, or derivatized nucleotide bases, D and/or L nucleoside enantiomers, protein nucleic acids (PNA), and the like. The backbone of the polynucleotide can comprise sugars and phosphate groups as may typically be found in RNA or DNA, or modified or substituted sugar or phosphate groups, including synthetic subunits such as phosphoramidites, and/or phosphorothioates (Peyrottes *et al.* (1996) *Nucl. Acids Res.* 24:1841-1848). Nucleic acids may also comprise modified nucleotides, such as methylated nucleotides and nucleotide analogs, uracyl, other sugars, and linking groups such as fluororibose and thioate, and nucleotide branches. The sequence of nucleotides can be interrupted by non-nucleotide components. Nucleic acids can be further modified by conjugation with a labeling component, addition of cap structures, introduction of means for attaching the polynucleotide to proteins, metal ions, labeling components, other polynucleotides, or a solid support, and other modifications as known in the art.

*Target nucleic acid.* A nucleic acid that contains a target nucleotide polymorphism of interest. Polymorphism refers to the occurrence of two or more genetically determined alternative sequences or alleles in a population. A polymorphic site is the locus at which divergence occurs. Polymorphisms may be substitution, addition or deletion of one or more nucleotides at a particular site. Frequently the detected variation will be a point mutation, or single nucleotide polymorphism. However, small deletions, additions, and multiple nucleotide variations are also detected.

The sample complexity, i.e. the length of sequence that will be analyzed, will usually be less than about 10¹⁰bp, more usually less than about 10⁹bp, and may be less than about 5 x 10⁷ bp in size. A viral genome will usually be greater than 10³ nucleotides in length, while a bacterial genome will usually be greater than 10⁵ bp in length. Larger genomes, e.g. having a complexity of greater than about 10⁷ bp, or greater than about 10⁸ bp, can optionally be separated into samples of lower complexity for analysis.

Target nucleic acids can be DNA, RNA transcripts thereof, or cDNA prepared from the RNA transcripts, but are usually genomic DNA. Target nucleic acids can comprise a plurality of different polymorphisms and can be derived from a variety of biological sources, including cell cultures, isolated cells, tissue samples, organs, and the like. Organisms of interest include single-celled organisms, *e.g.* fungus, such as yeasts; bacteria; viruses; protozoans; *etc.,* which organisms may be pathogens, organisms of agricultural interest, research models, *etc.* Multicellular organisms of interest include plants and animals, particularly mammals, including humans, non-human primates, cattle, sheep, goats, dogs, cats, birds (*e.g.*, chickens or other poultry), guinea pigs, rabbits, rats, mice, horses, and the like, where the physiological sources from multicellular organisms can be derived from particular organs or tissues of the multicellular organism, or from isolated cells derived therefrom.

The nucleic acid sample is prepared initially in accordance with conventional methods, *e.g.* lysing cells, removing cellular debris, separating the nucleic acid from proteins, lipids or other components present in the mixture and then using the isolated DNA for cleavage. See Molecular Cloning, A Laboratory Manual, 2nd ed. (eds. Sambrook et al.) CSH Laboratory Press, Cold Spring Harbor, NY 1989. The isolated DNA may be cleaved by digestion with restriction endonuclease or by physical manipulation, *e.g*. by repeatedly passing through a needle. Usually, at least about 0.5 µg of DNA will be employed, more usually at least about 5 µg of DNA, while less than 50 µg of DNA will usually be sufficient.

*Locus-specific primer pair.* A locus-specific primer pair is comprised of two oligonucleotides that hybridize to opposite strands of the target nucleic acid, and which hybridization sites flank the target polymorphism, with 3' hydroxyl ends of the primers facing each other. The exact composition of the primer sequences is not critical to the invention, but for most applications the primers will hybridize to the target nucleic acid sequence under stringent conditions, as known in the art. It is preferable to choose a pair of primers that will generate an amplification product (excluding the length contributed by the primers) of at least about 1 nt, and may be as much as 20 nucleotides, or 50 nucleotides in length, usually less than about 25000 nucleotides.

Known polymorphisms and their surrounding sequences are available in public databases including: SNP Database (The National Center for Biotechnology Information, National Library of Medicine, Bethesda, MD), Human Gene Mutation Database (see Krawczak and Cooper (1997) *Trends Genet.* 13:121-122), and Human Genic Bi-Allelic Sequences Database (Center for Genomics Research, Stockholm, Sweden; European Bioinformatics Institute, Cambridge, United Kingdom; European Molecular Biology Laboratory, Heidelberg, Germany), all of which are available via the World Wide Web. Algorithms for the selection of primer sequences are generally known, and are available in commercial software packages. Amplification primers hybridize to complementary strands of DNA, and will prime towards each other. The amplification product generated by the locus-specific primer pair will encompass the target polymorphism. The primer sequences are selected to be unique, *i.e.* the primer sequences are not complementary or identical to sequences found near other polymorphic sites, or to each other.

In referring to the primers, the terms "first locus-specific primer" and "second locus-specific primer" may be used, although those of skill in the art will appreciate that the designation is merely a matter of convenience. Each locus-specific primer is bound to the solid substrate at one end, thereby leaving a free end that is available for hybridization. Usually, the free end is the 3' end of the primer and the bound end is the 5' end of the primer. Preferably, the first and second primers are not spatially separated on the support, so that an amplification product can form between the first and second primers.

The primers can also comprise a spacer domain at the bound end. The spacer domain can be any convenient sequence, including random sequence or a non-polynucleotide chemical spacer (*e.g.* an ethylene glycol-based polyether oligomer). The spacer domain prevents steric hindrance from interfering with hybridization of the target sequence to the primer. Generally, the spacer domain if present, has a length ranging from about 1 to 20 subunits, usually from about 1 to 15 and more usually from about 1 to 10, including 5 to 10 subunits, where subunits may be nucleotides, amino acids, monosaccharides, *etc.* In one embodiment of the invention, one or both of the locus-specific primers has the structure: substrate-5'-S-H-3', wherein S is the spacer domain and H is the hybridization sequence.

In another embodiment, at least one of the locus-specific primers comprises an endonuclease recognition site, where the cognate endonuclease is selected to be an enzyme that cleaves at a site distant from its recognition site, and results in a cleavage product with staggered ends, *i.e.* the cleaved nucleic acid comprises a single-stranded overhanging strand and a recessed strand. Preferably, the recessed strand has a 3' terminus. A large number of such enzymes are known to those of skill in the art and are commercially available (sources include, for example, Stratagene; New England Biolabs; Promega; etc.). Specific endonucleases of use in the subject methods include, but are not limited to: Type IIS restriction endonucleases, homing endonucleases (*e.g.* as described in Belfort and Roberts, (1997) Nucleic Acids Research 25:3379-3388), and the like.

Type-IIS restriction enzymes interact with two discrete sites on double-stranded DNA: the recognition site, which is 4-7 bp long, and the cleavage site, usually 1-20 bp away from the recognition site. The recognition sequences of ENases-IIS are totally (or partially) asymmetric. Examples of Type IIS endonucleases for use in the subject methods include, but are not limited to: Alw XI, Bsm Al, Bsm Fl, Sts I, Hga I, Bsc Al, Bbv I, Bbv II, Bce fI, Bce 851, Bcc I, Bcg I, Bsa I, Bsg I, Bsp Ml, Bst 71 I, Ear I, Eco 571, Esp 31, Fau I, Fok I, Gsu I, Hph I, Mbo II, Mme I, RIe Al, Sap I, Sfa NI, Taq II, Tth 111II, Bco 51, Bpu Al, Fin I, Bsr DI, etc., and isoschizomers thereof. Preferred endonucleases include Fok I, and Bbv I.

Homing endonucleases are intron or intein encoded, and have a recognition sequence of 12-40 bp. The cleavage site leaves 3' and 5' overhangs of 1-10 bases. Examples of homing endonucleases include I-Ppo I; I-Ceu I; I-Dmo I; I-Sce I; PI-Sce I; PI-Psp I; and the like.

In primers where an endonuclease recognition site is present, the distance between such a recognition site, the targeted polymorphic site, and the hybridization site of the primer on the target nucleic acid are selected to provide for endonuclease cleavage at a position adjacent to the polymorphic site. In other words, the particular restriction endonuclease, the position of the hybridization site for the primer on the targeted nucleic acid, and the location of the endonuclease recognition site, are all selected to ensure that after amplification and endonuclease cleavage, the polymorphic nucleotide(s) reside on the single-stranded overhang of the cleavage product. One of skill in the art can readily determine the proper position of the recognition site within a primer based on the sequence of the recognition site, the distance downstream from the recognition site that the endonuclease cleaves a double-stranded nucleic acid, and the desired position of the polymorphic site on the single-stranded overhang of the cleavage product. Depending on the particular enzyme that is selected, the recognition site may be 5'; 3' or internal to the hybridization sequence; usually located 5' to the hybridization sequence.

In one aspect of this embodiment, the restriction endonuclease and location of sites are selected so that the first nucleotide on the single-stranded overhang of the cleavage product is a polymorphic nucleotide.

In another embodiment of the invention, at least one member of the locus-specific primer pair comprises a release recognition site. By "release recognition site" is meant a sequence that, when present as a duplex, is recognized and cleaved by an endonuclease that leaves blunt ends. Many such blunt-cutting endonucleases are known in the art, including but not limited to: Alul, Ball, EcoRV, HincII, Nrul, Smal, Stul, and the like. Depending on the particular enzyme that is selected, the release recognition site may be 5'; 3' or internal to the hybridization sequence, frequently located 5' to the hybridization sequence. When both primers comprise a release recognition site, it is preferable that different recognition sites are present on each primer, *e.g.* the same endonuclease will not recognize the release recognition site on both primers. In addition, the overhang left after digestion is preferably other than a 5' overhang, so that the release site provides for either a blunt end or a 3' overhang after digestion.

*Capture primer.* A single-stranded oligonucleotide comprising a capture sequence that is complementary to a locus specific sequence, or a DNA sequence found frequently in genomic DNA, such as repeated motifs, *e.g.* Alu sequence, LINES, SINES, and the like. Capture sequences are of a sufficient length to provide for specific hybridization to its complementary sequence. A capture primer can be from about 5 to about 50 nucleotides in length, but is generally at least about 10, and usually at least about 15 nucleotides in length and is generally not longer than about 40 and usually not longer than about 30 nucleotides in length. In one embodiment, the capture sequence can hybridize to substantially all target nucleic acids, i.e. at least about 80% of the target nucleic acids, more usually at least about 90% of the target nucleic acids, and preferably at least about 95% of the target nucleic acids. The capture primer is attached to the solid substrate at a location near the first and second locus-specific primers, so that the target nucleic acid can concurrently hybridize to both the capture primer and a locus-specific primer.

In one embodiment, the capture primer is attached to the solid substrate by a spacer domain, where such domains are as described above. The spacer domain attaching the capture primer to the substrate may be longer than the spacing domain, if any, attaching the first and/or second locus-specific primers to the substrate to further reduce steric hindrance of capture primer hybridization to target nucleic acid.

*Extension primer.* A nucleic acid that hybridizes to a site immediately adjacent to a targeted polymorphic site, but which does not extend over the polymorphic site. When the target nucleic acid and the extension primer are hybridized, the first unpaired base immediately downstream of the 3' end of the primer is the targeted polymorphic site. Preferably, an extension primer will only hybridize to one polymorphic site. In one embodiment of the invention, a plurality of extension primers are present in a hybridization mixture, where each extension primer hybridizes specifically to a different polymorphic site, i.e. under the conditions of the hybridization no extension primer binds to more than one site on target nucleic acids.

Where a plurality of extension primers are used, the extension primers may be preferably distinguishable by physical characteristics other than sequence, *e.g.* by position in a spatially addressable array, differences in length that provide for different mobilities during gel electrophoresis, *etc.* The polymorphic site corresponding to each primer can thus be identified by the position of the extension primer on the gel. Extension primers have a length sufficient to provide for unique hybridization, and are usually at least about 12 nt. in length, more usually at least about 14 nt. in length, and are usually not more than about 40 nt. in length. A range of sizes within this range may be used when multiple target polymorphisms are present in a sample.

*Labeled probe.* A specific binding member comprising a detectable label, which binds to the amplification product of a targeted nucleic acid, preferably binding to the polymorphic site. Preferred probes are nucleotides and nucleic acids, including polynucleotides, oligonucleotides, ribonucleotides, and single deoxynucleotides, dideoxynucleotides, ribonucleotides, dideoxyribonucleotides, *etc.* The probe can comprise a chain-terminating nucleotide, *e.g.* a dideoxynucleotide. A detectable label is linked to the binding member, which may be directly or indirectly detectable, preferably directly detectable. Usually one or more nucleotide residues are modified to include a label, where the modified residue may be a chain terminating nucleotide.

Directly detectable labels include isotopic labels, in which one or more of the nucleotides is labeled with a radioactive label, such as ³²S, ³²P, ³H, *etc.* Fluorescent labels of interest include: fluorescein, rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), the cyanine dyes, such as Cy3, Cy5, Alexa 542, Bodipy 630/650, fluorescent particles, fluorescent semiconductor nanocrystals, and the like. The labeled probes can be produced using any convenient protocol.

In one embodiment, the labeled probe is a single-stranded oligonucleotide complementary to the single-stranded overhang of the cleavage product resulting from cleavage by the distance-cleaving endonuclease, where the probe may consist solely of such a complementary sequence, or may extend beyond the complementary sequence.

In another embodiment, the probe comprises a chain-terminating nucleotide, *e.g.* dideoxyadenosine triphosphate (ddATP), dideoxycytosine triphosphate (ddCTP), dideoxythymidine triphosphate (ddTTP), dideoxyguanosine triphosphate (ddGTP), dideoxyuridine triphosphate (ddUTP), *etc.*

By *"solid substrate" or "solid support"* is meant any surface to which the nucleic acid primers of the subject invention are attached. A variety of solid supports or substrates are suitable for the purposes of the invention, including both flexible and rigid substrates. By flexible is meant that the support is capable of being bent, folded or similarly manipulated without breakage. Examples of flexible solid supports include nylon, nitrocellulose, polypropylene, polyester films, such as polyethylene terephthalate, *etc.* Rigid supports do not readily bend, and include glass, fused silica, quartz, acrylamide; plastics, *e.g.* polytetrafluoroethylene, polypropylene, polystyrene, polycarbonate, and blends thereof, and the like; metals, *e.g*. gold, platinum, silver, and the like; *etc.*

The substrates can take a variety of configurations, including filters, fibers, membranes, beads, particles, dipsticks, sheets, rods, *etc.* The materials from which the substrate can be fabricated should ideally exhibit a low level of non-specific binding during hybridization events.

In one embodiment of the invention, the substrate comprises a planar surface, and the primers are spotted on the surface in an array, with both the first and second locus-specific primers present in the same primer spot. The substrate can comprise a plurality of primer spots so that a plurality of locus-specific primer pairs are bound to the array. The primer spots on the substrate can be any convenient shape, but will often be circular, elliptoid, oval or some other analogously curved shape. The density of the spots on the solid surface can be at least about 5/cm² and usually at least about 10/cm² but does not exceed about 1000/cm², and usually does not exceed about 500/cm², and more usually does not exceed about 300/cm². The spots can be arranged in any convenient pattern across or over the surface of the support, such as in rows and columns so as to form a grid, in a circular pattern, and the like, where generally the pattern of spots will be present in the form of a grid across the surface of the solid support.

The total number of primer spots on the substrate will vary depending on the number of different targeted polymorphic sites, as well as the number of control spots, calibrating spots and the like, as may be desired. Generally, the pattern present on the surface of the support will comprise at least about 10 distinct spots, usually at least about 20 distinct spots, and more usually at least about 50 distinct spots, where the number of spots can be as high as 50,000 or higher, but will usually not exceed about 25,000 distinct spots, and more usually will not exceed about 15,000 distinct spots. Each distinct probe composition may be present in duplicate to provide an internal correlation of results.

In another embodiment, the substrate is a collection of physically discrete solid substrates, *e.g.* a collection of beads, individual strands of fiber optic cable, and the like (see , for example, U.S. Patent nos. 6,023,540; 5,814,524; 5,633,972; and 5,512,490). Each discrete substrate can have one or more primer spots covalently attached to the surface of the bead. The collection of physically separable discrete substrates may be arranged in a predetermined pattern so that the location of each locus-specific primer pair is known. Alternatively, labels, *e.g.* fluorescent tags, may be present as an identifier on the substrates.

The amount of primer present in each spot will be sufficient to provide for adequate hybridization and detection of target nucleic acid during the assay in which the array is employed. Where the spot has an overall circular dimension and the diameter of the spot is ranging from about 10 to 5,000 µm, usually from about 20 to 2,000 µm and more usually from about 50 to 1000 µm, then the amount of polynucleotide in each spot will be at least about 0.1 ng, usually at least about 0.5 ng and more usually at least about 1 ng, where the amount can be as high as 1000 ng or higher, but will usually not exceed about 20 ng and more usually will not exceed about 10 ng. The copy number of each first and second locus-specific primer in a spot will be sufficient to provide enough hybridization sites for target nucleic acid to yield a detectable signal, and will generally range from about 0.01 fmol to 50 fmol, usually from about 0.05 fmol to 20 fmol and more usually from about 0.1 fmol to 5 fmol.

The subject substrates can be prepared using any convenient means. One means of preparing the supports is to synthesize the locus-specific primers, and then deposit as a spot on the support surface. The primers can be prepared using any convenient methodology, such as automated solid phase synthesis protocols, preparative PCR and like, where such techniques are known in the art. The prepared primers can then be spotted on the support using any convenient methodology, including manual techniques, *e.g.* by micro pipette, ink jet, pins, etc., and automated protocols. Of particular interest is the use of an automated spotting device, such as the Beckman Biomek 2000 (Beckman Instruments). Alternatively, the primers can be synthesized on the substrate using standard techniques known in the art.

### METHODS FOR DETERMINING SEQUENCE POLYMORPHISMS

The subject invention provides a high throughput method for detecting nucleotide polymorphisms in a complex mixture of nucleic acids where one or more distinct polymorphisms can be present in the mixture, and multiple polymorphisms can be screened in parallel. The subject methods have many uses, including diagnosis of disease or genetic predisposition to disease, determination of genetically controlled differences in metabolism of therapeutic agents, and response to therapeutic agents.

Genes with polymorphisms known to be associated with disease include: IL6 (Fishman (1998) *J. Clin. Invest.* 102, 1369), blnk (Minegishi (1999) *Science* 286, 1954), TM4SF2 (Zemni (2000) *Nat. Genet.* 24, 167), SLC6A2 (Shannon (2000) *N. Engl. J. Med.* 342, 541), SCN1A (Escayg (2000) *Nat. Genet.* 24, 343), PIK3R1 (Oldridge (2000) *Nat. Genet.* 24, 275), NR2E3 (Haider (2000) *Nat. Genet.* 24, 127), MAPK8IP1 (Waeber (2000) *Nat. Genet.* 24, 291), IL1B (EI-Omar (2000) *Nature* 404, 398), HNF4B (Yamada (2000) *Diabetologia* 43, 121), GJB6 (Grifa (1999) *Nat. Genet.* 23, 16), EVC (Ruiz-Perez (2000) *Nat.* Genet. 24, 283), CX3CR1 (Faure (2000) *Science* 287, 2274), CTSD (Papassotiropoulos (2000) *Ann. Neurol.* 47, 399), B4GALT7 (Okajima (1999) *J. Biol. Chem.* 274, 28841), AMACR (Ferdinandusse (2000) *Nat. Genet.* 24, 188), ACTN4 (Kaplan (2000) *Nat. Genet.* 24, 251), and SDHD (Baysal (2000) *Science* 287, 848). Additional disease-associated genes can be found in publicly available databases such as the SNP Database, Human Gene Mutation Database, and Human Genic Bi-Allelic Sequences Database.

The subject methods can also be used to analyze polymorphisms for correlation with a particular trait. A set of polymorphisms is analyzed for a set of individuals, some of which exhibit a particular trait, and some of which do not (e.g. diagnosed with Alzheimer's disease vs. no Alzheimer's disease). The alleles of each polymorphism in the set are then reviewed to determine whether the presence of absence of a particular allele is associated with the particular trait of interest. Alleles that do not segregate randomly with respect to the trait can be used to predict the probability that a particular individual will express that trait. Such methods can also be used to construct a genetic map of a particular species.

In the subject methods one or more target nucleic acids comprising target polymorphic sites is amplified by a bridge amplification method. A sample comprising the target nucleic acids is denatured, if not already single-stranded, and placed in contact with solid substrate comprising one or locus-specific primer pairs, as described above, under conditions that provide for specific hybridization of the primers. An amplification method of particular interest in the subject methods is that described in U.S. Patent No. 5,641,658, the disclosure of which is incorporated herein by reference.

The first locus-specific primer and target nucleic acid regions of complementarity are hybridized and the first locus-specific primer is used to prime a DNA polymerase to extend the primer sequence, using the target nucleic acid as a template. The extension product and the target nucleic acid are denatured, and allowed to hybridize again to the primers present on the solid substrate. The second locus-specific primer hybridizes to the first strand, and is used to prime the reverse strand.

This double-stranded amplification product is then analyzed for the identity of the polymorphic nucleotide, through methods that covalently attach at least one labeled nucleotide to the amplification product, where the labeled nucleotide is selected by it's hybridization properties. The bond that is formed between the nucleotide and the amplicon is catalyzed by an enzyme, e.g. DNA polymerase, DNAligase, that utilizes such specificity.

For methods using DNA polymerization as a readout, the amplicon can be denatured to yield two single stranded products comprising a first and a second locus-specific primer, respectively, where both are attached to the solid support. An extension primer, as described above, is then hybridized to one of the strands, such that the nucleotide base to be identified in the polymorphic site is the first unpaired base immediately downstream of the 3' end of the primer. The extension primer is hybridized to the amplification product under stringency conditions such that an exact match between the extension primer and the amplification product is obtained without any base-pair mismatches.

In another primer extension protocol, the amplification product provides an internal extension primer to provide a template for DNA synthesis. A distance endonuclease recognition site is positioned within the primer so that the first nucleotide on a single-stranded overhang of the cleavage product is a polymorphic nucleotide, where a one nucleotide extension of the 3' terminus of the recessed strand will base pair with the polymorphic nucleotide. In this embodiment, a release recognition cleavage providing either a blunt or 3' overhang endonuclease is also used.

The duplex formed by the extension primer and complementary sequence is contacted with a labeled probe that will base pair with the nucleotide in the polymorphic site. The labeled probes can be two, three or four differentially labeled dideoxynucleotides, *e.g.* ddATP, ddCTP, ddGTP, ddTTP, ddUTP, *etc.* An enzyme that will prime from the extension primer, *e.g.* a DNA polymerase is added, so that a labeled dideoxynucleotide is covalently incorporated into the 3' end of the extension primer. Because extension by the polymerase depends on correct base pairing at the polymorphic site, the identity of the incorporated dideoxynucleotide(s) is complementary to the nucleotide present at the polymorphic site. The identity of the detectable label at the 3' end of the extension primer thereby indicates the complement of the nucleotide in the polymorphic site.

The extension product can be analyzed from the observed label pattern *in situ* while hybridized to the substrate bound amplification product. Alternatively, where mutually distinguishable extension primers are used, *e.g.* primers of different lengths, the extension primers can be denatured and separated by gel electrophoresis, and the gel analyzed. The labels can be detected by conventional methods known in the art. For example, where the labels are fluorescent labels, a laser excitation source can be used in conjunction with a filter set to isolate the fluorescence emission of a particular label, which corresponds to one of the dideoxynucleotides. A photomultiplier tube, charged-coupled device (CCD), or another suitable fluorescence detection method can be used to detect the emitted light from fluorescently labeled probes. The label and position of each probe either within the substrate or on the gel directly defines the sequence of the polymorphic site of the target nucleic acid.

In methods using a ligase readout, a first locus-specific primer comprises an endonuclease recognition site for cleavage at a distance, and at least one of the first and second locus-specific primers comprises a release recognition site. The double-stranded amplification product is linearized by contacting with an endonuclease, thereby releasing one end of the amplification product from the substrate as a free end. The release endonuclease should not provide an overhang that is the same as the overhang containing the polymorphism.

The linearized amplification product is then contacted with a distance-cleaving endonuclease that cleaves the amplification product such that the polymorphic site is on the single-stranded overhang of the cleavage product. The cleavage product remains attached to the solid support, and the substrate can then be washed to remove the fragment released by the endonuclease.

The substrate-bound cleavage product can then be contacted with a labeled probe, where one, two, three, or four differentially labeled probes are used, and each distinguishable label corresponds to a particular nucleotide that will base pair with the nucleotide in the polymorphic site.

In one embodiment, the substrate-bound cleavage product is hybridized with a mixture of labeled oligonucleotide probes of the same length as the single-stranded overhang of the cleavage product under stringency conditions so that an exact match between the single-stranded overhang of the cleavage product is obtained without any base-pair mismatches. For example, if the single-stranded overhang is four nucleotides in length and four different labels are used to represent the four possible nucleotides in the polymorphic site, 256 probes are needed to represent all possible combinations of the four nucleotides, *i.e.* each label will be present on 64 probes which contain all possible combinations of the three overhang positions along with fixed 4^{th} position in the polymorphic site. Preferably, three or four distinguishable labels are used, in which case the probe mixture comprises 192 or 256 probes.

The probes are hybridized under conditions that only permit perfect matches to base-pair to the single-stranded overhang. The hybridized probes are ligated to the cleavage product, thereby forming a covalent bond, and stringently washed to remove unhybridized and/or unligated probe. The identity of the label indicates which nucleotide in the oligonucleotide probe has hybridized to the nucleotide present in the polymorphic site.

The labels can be analyzed while on the substrate in situ from the observed label pattern. Alternatively, the labeled cleavage product is released from the substrate by contacting the cleavage product with a second blunt-cutting endonuclease which cleaves the cleavage product at a release recognition site, thereby releasing the labeled cleavage product from the substrate for analysis. In this embodiment, the locus-specific primers of the cleavage product are mutually distinguishable, *e.g.* by having different mobilities during gel electrophoresis from primers of different length. The labeled and unlabeled strands of the cleavage product are denatured and used for gel electrophoresis. The labeled strand of the cleavage product can also be separated from the unlabeled strand by denaturing gel electrophoresis. The nucleotide in the polymorphic site is determined from the label pattern observed on the gel.

### KITS

Also provided by the subject invention are kits for use in determining the identityof one or more polymorphic nucleotide(s) in a nucleic acid sample. The kits may comprise containers, each with one or more of the various reagents utilized in the methods, including, for example, dNTP's, polymerase, labeled probes, capture primers, extension primers, *etc.* In addition, the kits can also comprise a plurality of locus-specific primer pairs, where the primer pairs may be bound to one or more solid substrates, *e.g.* an array or collection of physically discrete solid substrates. The kits can further comprise a set of instructions, where the instructions may be associated with a package insert and/or the packaging of the kit or kit components.

### EXPERIMENTAL

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1

### Materials and Methods:

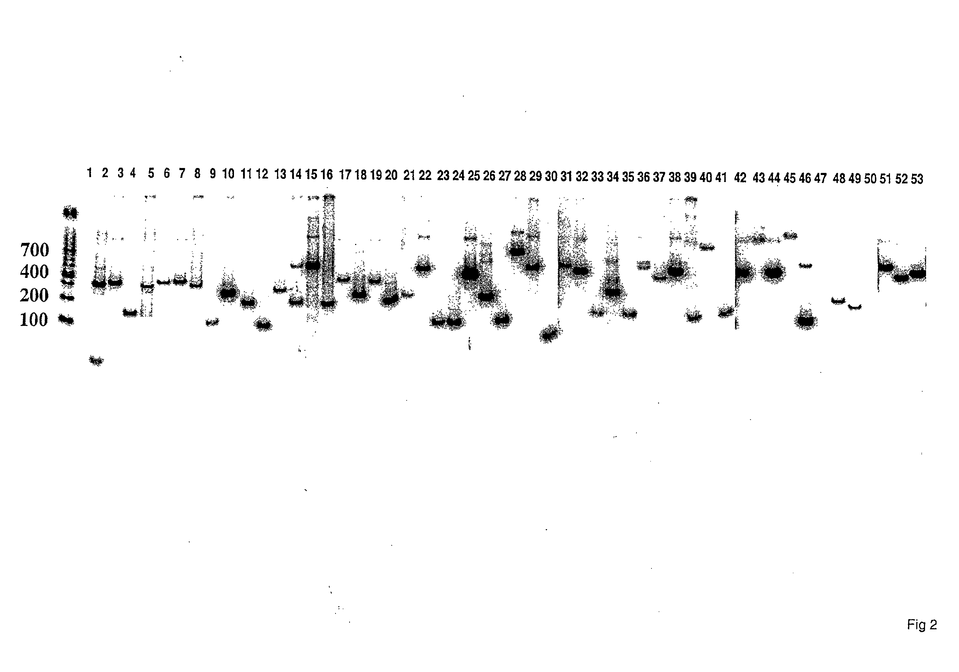

Oligonucleotide Synthesis. Oligonucleotides with a 5' acrylamide group were obtained from Operon Technologies, Inc. (Alameda, CA). This modification was incorporated during automated synthesis using an acrylamide phosphoramidite (Acrydite™; Mosaic Technologies, Boston, MA). Primer sequences for multiplexed amplification shown in Figure 2 are available on request. Lyophilized oligonucleotides were resuspended at a concentration of 1 mM with 1X TE (10 mMTris-HCI, pH 8.0, 1 mM EDTA) and were stored frozen at -20°C.

Preparation of acrylamide beads: Gel solutions contained 10% Acrylamide (29:1 w/w acrylamide: bis-acrylamide), 10mM sodium borate buffer, 0.2% ammonium persulfate, and 100 µM of each Acrydite™ primer. Nitrogen-saturated mineral oil containing 0.4% TEMED (N, N, N', N'-tetramethylenediamine) was placed into a small polyethylene dish (weighboat), and 1.0 or 0.5 µl aliquots of the gel solution were pipetted under the mineral oil. Beads were polymerized at room temperature for 1 hour. The mineral oil was decanted and the beads were recovered in TE.

Beads were loaded into large wells of a 2% Agarose gel in 0.5x TBE, and unpolymerized primer was removed by electrophoresis at 130V for 1 hour. Beads were removed from the wells with a large bore pipet tip, and washed in TE. Beads were stored in TE at 4°C for up to 4 weeks. Beads were pre-cycled to decrease non-specific amplification (94°C for 10 min., then 94°C for 45 sec, 45°C for 1 min., 72°C for 1 min. for 15 cycles) in 1x GeneAmp PCR buffer (10 mM Tris-HCl, pH 8.3, 50 mM KCI, 1.5 mM MgCl₂, 0.001 % (w/v) gelatin) containing 0.25 ng/µl *E. coli* genomic DNA (Sigma). Beads were washed 3 times in STE (TE containing 50 mM NaCI), and twice in 1x GeneAmp PCR buffer prior to target capture.

Target hybridization and solid-phase amplification. For target hybridization, beads were incubated in 1x GeneAmp or 1x Taq Extender (Stratagene) buffer (20 mM Tris-HCl pH 8.8, 10 mM KCI, 10 mM (NN₄)₃SO₄, 2 mM MgSO₄, 0.1% Triton X-100, 0.1 mg/ml bovine serum albumin) containing 100-125 ng/µl denatured, human genomic DNA that had been either sheared or digested with Stul. Target capture volumes were adjusted to just cover the beads (10 µl for a single 1 µl bead, 80 to 100 µl for 50 1 µl or 100 0.5 µl beads). Human genomic DNA was sheared to a 2 to 5 kb average size by repeated passing through a 261/2 gauge needle. Genomic DNA was denatured by heating at 95°C for 10 minutes, followed by quenching on ice.

Beads were hybridized with human genomic DNA for 12 to 24 hours in an Eppendorf Thermomixer at 45°C and 850 rpm. After target capture, reactions were washed twice in STE, and twice in 1x Taq Extender buffer. Solid-phase amplification reactions contained 1x Taq Extender buffer, 200 µM of each dNTP, 2.5U Amplitaq, 5U AmplitaqTaq Gold, and 5U Taq Extender. In cases where PCR products were visualized directly, 1 µl (10 µCi) of ³²P-α-dCTP (3000 Ci/mmole) was also included. Reactions underwent an initial extension protocol (60°C for 5 min., 68°C for 5 min., 72°C for 10 min.) prior to cycling (94°C for 10 min., then 94°C for 45 sec, 65°C -1 °C/cycle for 1 min., 72°C for 1 min. for 20 cycles, followed by 94°C for 45 sec, 45°C for 1 min., 72°C for 1 min. for 70 cycles). After cycling, beads were washed 5 times in STE.

Release of ³²P-labeled solid-phase PCR product. Single beads were incubated for 12 to 16 hours in 10 µl of solution containing appropriate restriction enzymes. Supernatants were loaded onto a 10% Acrylamide TBE (89 mM Tris-borate, 89 mM Boric acid, 2 mM EDTA) gel and run at 200V for 1 hour. Gels were dried and exposed to phosphoimaging cassettes for 1 to 3 hours. Screens were scanned with a Molecular Dynamics Storm 860 instrument at 200 µm resolution.

One-Color SNP Minisequencing. Individual beads used to test Type IIS enzymes contained the primer 5'-QTTTTTTGATATCGCAGCAGAGGGACTGGAGAAGCATACACTTCTGAT-3' (SEQ ID NO: 1), where Q designates the acrylamide group. A complementary oligonucleotide 5'-ATCAGAAGTGTGTATGCTTCTCCAGTCCCTCTGCTGCGATATCAAAAAA-3' (SEQ ID NO: 2) was annealed to form the duplex. Bbvl and single color minisequencing reactions were carried out as described below, except that products were released by digestion with 10 U EcoRV at 37°C for 16 hr and resolved on 20 % 1X TBE acrylamide gels.

Individual beads containing primers for a polymorphism in the human 5-hydroxytryptamine type 2a receptor (5-HT₂ₐ) were used for solid-phase amplification.The acrydite primers were 5'-T₁₂AGGCCTACACCAGGCTCTACAGCAGCGACTTTAACT-3' (SEQ ID NO: 3) (FW) and 5'-T₁₂CAGCTGGGCACCCTTCACAGGAAAGGTTGGTTCG-3' (SEQ ID NO: 4) (RV). After amplification, beads were equilibrated into 1xNEBuffer 2 (New England Biolabs; 50 mM NaCI, 10 mM Tris-HCl pH 7.9 @25°C, 10 mM MgCl₂, 1 mM DTT, and digested at 37°C for 16 hr with 10 U Stul per bead. Beads were then rinsed with 1x NEBuffer 2, and incubated for 4 hr at 37°C with 1 U*Bbv*I per bead. Beads were rinsed and equilibrated into 1x Amplitaq FS buffer for 10 min at room temperature.

One-color sequencing reactions contained 1x Amplitaq FS buffer, 1.5 µM of the single FAM (5-carboxyfluorescein)-labeled ddNTP (ddATP, ddCTP, ddGTP, or ddUTP) as well as 1.5µM of each of the three unlabelled ddNTPs. Reactions were incubated at 68°C for 30 minutes, and then beads were rinsed twice with TE buffer and unincorporated fluorescent ddNTP was removed by electrophoresis of the beads at 130 V for 1 hour. Beads were equilibrated into 1x NEBuffer 2, and digested overnight at 37°C with 10 U *Pvu*II enzyme. Released products were analyzed by electrophoresis on 10% Acrylamide, 1x TBE gels and run at 200V for 1 hour. Gels were scanned with a Storm 860 instrument (Blue fluorescence filter, PMT 900 V) at 200 µm resolution to detect the fluorescent signal. Gels were then stained with Sybr Green I (Molecular Probes) diluted 1:10,000 in 1x TBE for 30 min and re-imaged using the same instrument setup to detect total released PCR product in all lanes.

4-Color SNP Sequencing. Solid-phase amplification reactions, each containing 8 beads, were equilibrated into 1x NEBuffer 2 and digested overnight at 37°C with 40 U *Stu*I restriction enzyme. Beads were washed once with fresh 1xNEBuffer 2, and then digested overnight at 37°C with 10 U of the Type IIS enzyme*Bbv*I. Reactions were then equilibrated into 1x Amplitaq FS buffer. 4-color sequencing reactions (100 µl) contained 1x Amplitaq FS buffer, 2 mM Manganese Citrate, 1.5 µM R6G-ddATP, 1.5 µM R110-ddGTP, 1.5 µM ROX (6-carboxy-X-rhodamine)-ddUTP, 1.5 µM TAMRA (N, N, N', N'-tetramethyl-6-carboxyrhodamine)-ddCTP, and 5 U Amplitaq FS. All fluorescently labeled ddNTPs were from NEN Life Science Products.

Reactions were incubated at 68°C for 30 minutes, rinsed twice with TE buffer, and unincorporated fluorescent ddNTPs were removed by electrophoresis of the beads at 130V for 1 hour. Beads were equilibrated into 1x NEBuffer 2, and digested overnight at 37°C with 40 U *Pvu*II enzyme. Reaction supernatants were collected, adjusted to 0.3 M NaOAc pH 5.3, and 20 µg glycogen carrier was added. Products were precipitated with the addition of 2.2 volumes ethanol and centrifugation at 14,000 rpm for 30 min, washed with 70 % ethanol, and then air-dried. Reactions were resuspended in 10 µl TE buffer. Aliquots were mixed with an equal volume of loading solution (5:1 deionized Formamide: 50 mM EDTA), heated at 90°C for 2 min followed by an ice quench, and run on 6% acrylamide (19:1 acrylamide: bis-acrylamide), 8.3 M urea, 1x TBE gels using a 36 cm well-to-read distance. An Applied Biosystems (ABI) 373 DNA Sequencer (STRETCH) instrument was used together with ABI 672 GeneScan Collection Software (version 1.1) and GeneScan PCR Analysis Software (version 1.2.2-1). A multicomponent matrix was generated using Taq DyeDeoxy Terminator Matrix Standards (ABI).

### Multiplexed Solid-phase PCR with Human Genomic DNA

Bead-bound primers (Rehman, et al. (1999) *Nucl. Acids Res.* 27:649-655) for multiplexed solid-phase PCR contain a 12 base spacer, a NotI site (8 bases), and 18 to 33 bases of locus-specific sequence. Solid-phase PCR was performed in the presence of ³²P-α-dCTP; beads were rinsed, and then incubated for 12 to 16hours with *Not*I to release reaction products. Reaction products were resolved on 10% 1X TBE acrylamide gels and imaged with a STORM 860 phosphoimager.

It was established that specific PCR products could be generated directly from human genomic DNA as template on acrylamide beads bearing covalently attached primer pairs. To evaluate whether solid-phase PCR was capable of handling the high-throughput required for genotyping, PCR reactions containing multiple 1.0µl acrylamide beads were performed. Figure 2 shows reaction products from 53 beads bearing different primer pairs. The 53 reaction products range in size from approximately 70 bp to 1300 bp, and represent genes from 16 different chromosomes. Multiplexing of up to 102 beads was also demonstrated (17 distinct amplicons each represented 6 times).

### EXAMPLE 2

### Single Color SNP Minisequencing

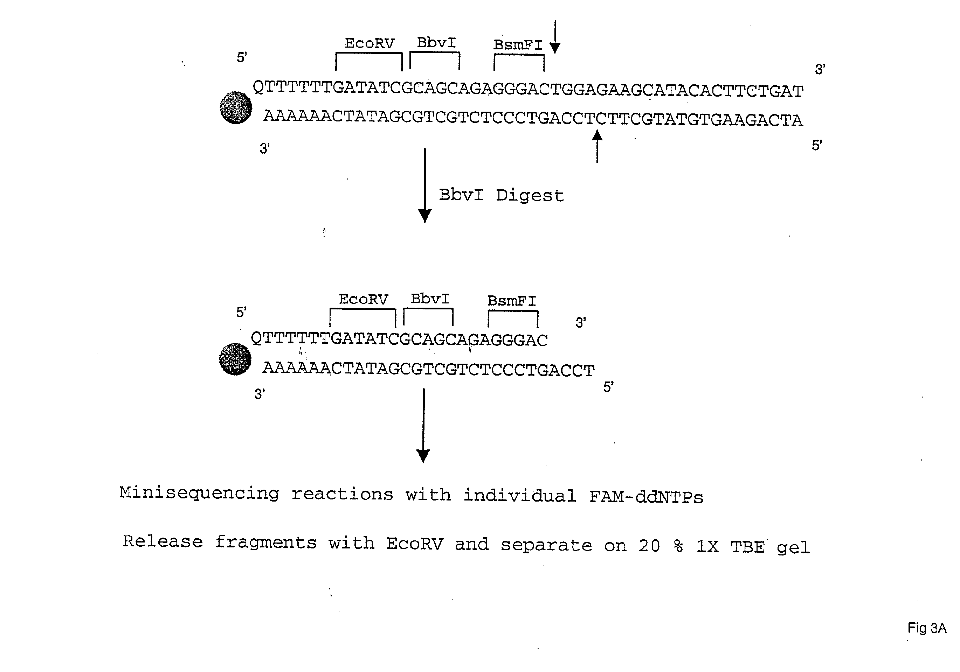

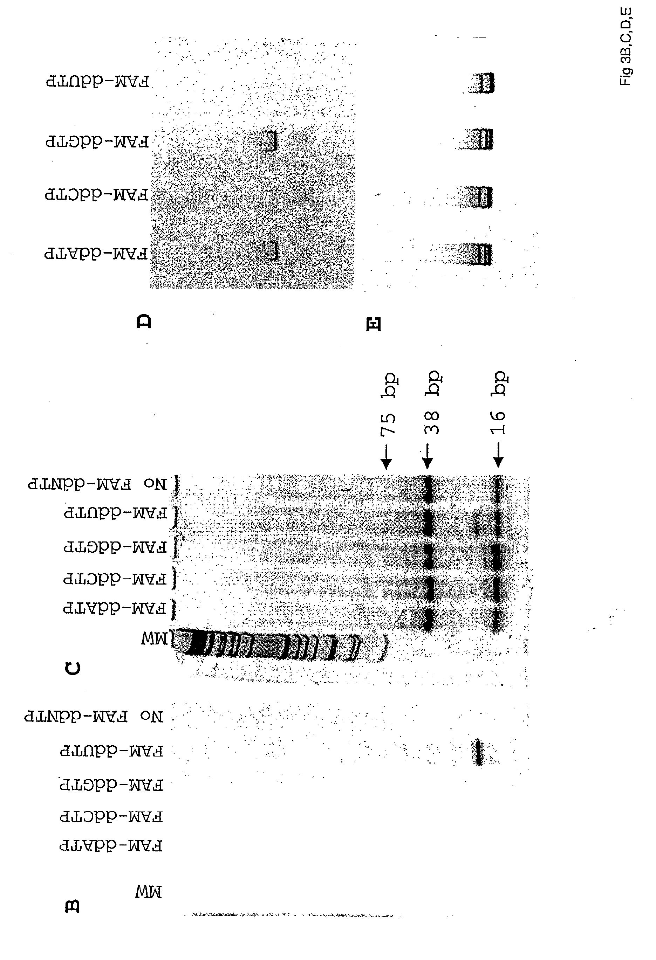

The genotyping approach required a Type IIS restriction enzyme site to be precisely positioned within one of the PCR primers. In order to determine the accuracy of various Type IIS enzymes, artificial DNA duplexes were prepared on beads and used as enzyme substrates. Figure 3A shows the sequence of a duplex containing recognition sites for *Bbv*I and *BsmF*I. The expected sequence of the duplex following *Bbv*I digestion is also shown in Figure 3A. These digested duplexes were used as templates in minisequencing reactions with individual FAM-ddNTPs. Reaction products were released by EcoRV digestion and resolved on 20 % 1X TBE acrylamide gels. The fluorescent gel image (Figure 3B) shows only the expected nucleotide FAM-ddUTP was incorporated into the duplex. Sybr Green I staining (Figure 3C) of the gel shows equal amounts of digested duplex was released from all the beads. Thus *Bbv*I accurately cleaves an artificial duplex immobilized to the surface of acrylamide microspheres.

To assess the specificity of the genotyping scheme, a 135 bp amplicon spanning the T102C polymorphism in the human gene for the 5-hydroxytryptamine type 2a (5-HT₂ₐ) receptor (Warren, *et al.* (1993) *Hum. Mol.* Genet. 2:338) located on chromosome 13q was evaluated using a single color SNP minisequencing format. There are several general features of the primer design for solid-phase amplification and genotyping. Each primer-pair designed for a specific polymorphic locus introduced a *Stu*I site and a *Pvu*II site into the resulting PCR product. In addition, the 6 bp Type IIS *Bbv*I restriction enzyme site, embedded within locus-specific sequence, was always positioned 12 nucleotides away from the SNP, either in the forward primer or in the reverse primer. The site was placed in the forward primer for the 5-HT₂ₐ model amplicon, resulting in ddNTP incorporation into the noncoding strand. The *Bbv*I site was used only in conjunction with the *Stu*I site. *Stu*I digestion linearized the bound PCR product generating blunt ends that could not serve as templates for ddNTP incorporation. *Bbv*I digestion of the linearized product released a short segment containing the *Bbv*I recognition sequence, as well as exposed the polymorphic nucleotide in a 5' overhang on a fragment attached to the bead. Extension of the 3'hydroxyl group of the recessed nucleotide with a single fluorescentddNTP led to incorporation at the position of genetic variation.

Fluorescently labeled fragments were released from the beads by cleavage with *Pvu*II. Individual beads containing a 5-HT₂ₐ primer-pair were used for target hybridization and solid-phase amplification with genomic DNA template from a single individual. Restriction enzyme digests with *Msp*I and *Bpm*I in addition to direct sequencing of 5-HT₂ₐ PCR products confirmed the genotype as a C/T heterozygote. Following *Stu*I and *Bbv*I cleavage, beads were used in minisequencing reactions with individual FAM-ddNTPs. Products released by *Pvu*II digestion are shown in Figure3D. Fluorescent signal is observed only from the FAM-ddATP and FAM-ddGTP labeling reactions, corresponding to incorporation into the opposite strand of the T and C polymorphisms. Sybr Green I staining of the gel after fluorescent imaging (Figure 3E) shows that equal amounts of 5-HT₂ₐ PCR product were synthesized and released from each bead. The results show that *Bbv*I can accurately cleave a linearized solid-phase amplification product, which can then serve as a template for ddNTP incorporation.

### EXAMPLE 3

### Four Color SNP Minisequencing

Single color minisequencing reactions were converted to 4-color minisequencing reactions by using ddNTPs that were individually labeled with distinct rhodamine dyes. Fluorescent products were enzymatically released from beads, separated by denaturing gel electrophoresis and data collection and analysis was via an automated sequencer with GeneScan software. Incorporation of the rhodamine dye-labeled ddNTPs resulted in a gel image that was blue for R110-ddGTP, red for ROX-ddUTP, green for R6G-ddATP, and yellow for TAMRA-ddCTP. This platform therefore permitted quantitative detection of any single color (homozygous genotype) or any two-color combination (heterozygous genotype) in an amplification product. Additionally, any SNPs that were not bi-allelic could also be detected with this system.

Multiplexing of the solid-phase amplification and minisequencing reactions was achieved by designing each released product to have a specific, discrete length. Thus the length of the detected product identified the polymorphic locus. Eight polymorphic loci were used to assess the accuracy of the multiplexed genotyping scheme. The sequences of the primers used are shown in Table 1 below.

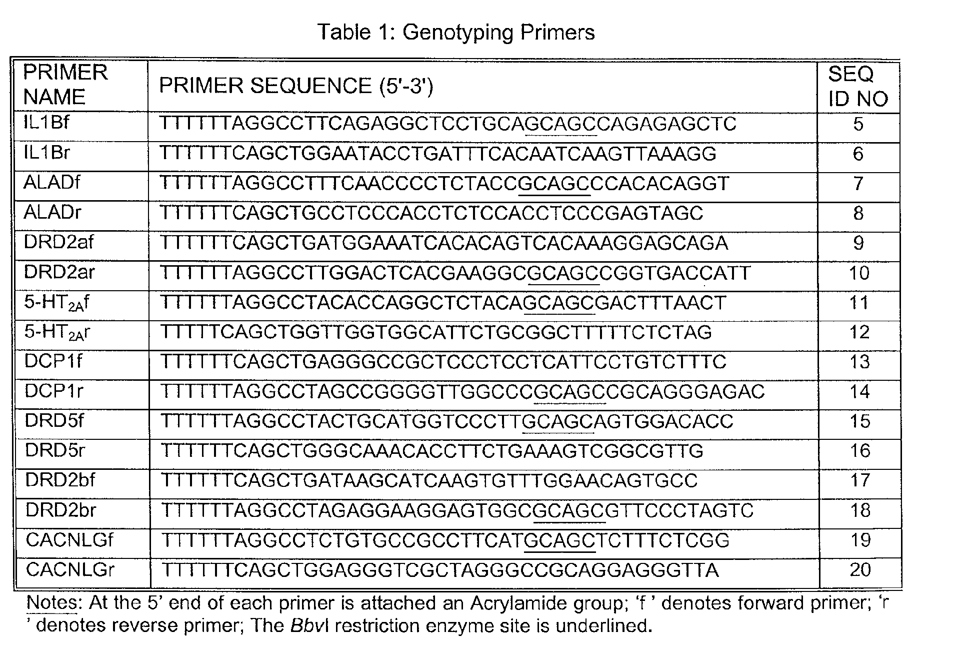

Each human locus used for analysis is listed with the polymorphism and restriction enzyme used for standard genotyping. They are listed in order of length (longest to shortest) of the released products: interleukin-1B (IL1B; Aval; C/T) (Di Giovine, *et al.* (1992) *Hum. Mol. Genet.* 1:450), δ-aminolevulinate dehydratase (ALAD; Rsal; C/T) (Astrin, *et al.* (1991) *Nucl. Acids Res.* 19:4307), dopamine D2 receptor (DRD2a; HincII; C/G) (Wetmur, *et al.* (1991) *Am. J. Hum.* Genet. 49:757-763), 5-hydroxytryptamine type 2a receptor (5-HT2a; Mspl; T/C) (Hauge, *et al.* (1991) *Genomics* 10:527-530), dipeptidyl carboxypeptidase (angiotensin I converting enzyme) (DCP1; BsmBI; T/C) (Reider, *et al.* (1999) *Nat. Genet.* 22:59-62), dopamine D2 receptor (DRD2b; TaqI; C/T) (Wetmur, *et al.* (1991) *Am. J. Hum.* Genet. 49:757-763), D5 dopamine receptor (DRD5; Eco57I; C/T) (Sommer, *et al.* (1993) *Hum. Genet.* 92:633-634), and they subunit of the L-type Ca²⁺ channel (CACNLG; Acil; G/A) (Olckers, *et al*. (1993) *Hum. Mol. Genet.* 2:2198).

The *Bbv*I recognition site was placed in the forward primer in the ALAD, 5-HT₂ₐ, IL1B, DRD5, and CACNLG amplicons. This resulted in fluorescent labeling of the noncoding strand. Therefore, blue electropherogram tracings indicated a C allele, red tracings indicated an A allele, green tracings indicated a T allele, and black (yellow) tracings indicated a G allele. The *Bbv*I recognition site was placed in the reverse primer for the DRD2a, DRD2b, and DCP1 amplicons. This resulted in fluorescent labeling of the coding strand. Therefore, blue electropherogram tracings indicated a G allele, red tracings indicated a T allele, green tracings indicated an A allele, and black (yellow) tracings indicated a C allele.

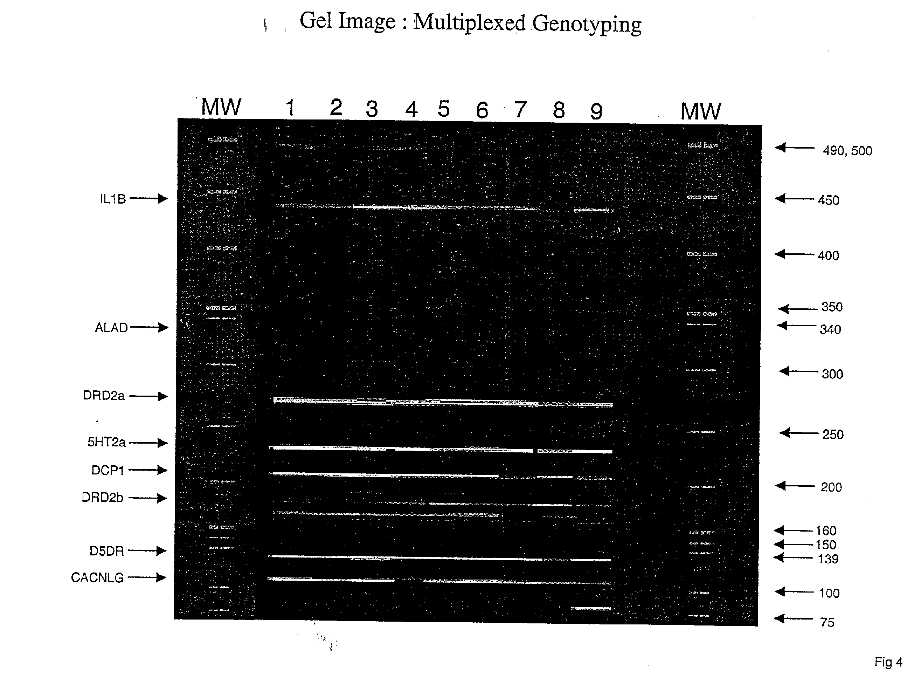

Bead sets for the eight polymorphic loci were used for target hybridization and multiplexed solid-phase amplification with genomic DNA from eight different individuals. These individuals had previously been genotyped at each of the polymorphic loci by restriction enzyme digestion of PCR products, and in some instances by direct DNA sequencing of PCR products. Following multiplexed solid-phase PCR, each bead set was used for 4-color SNP minisequencing. Fluorescent-ddNTP incorporation reactions were carried out directly on bead-bound products. This allows conversion to a format in which the SNP genotype is readout directly on an optically encoded bead population. Labeled products were released by *Pvu*II digestion, and separated on a 6 %, 8 M urea acrylamide gel. A representative GeneScan gel image is shown in Figure 4.

Identification of the released products, whose lengths encode the specific polymorphic loci being analyzed, was determined by comparison to a GeneScan-500 ROX size standard and to an internal reference standard. The eight displayed products for each individual span a range of sizes from ∼ 420 b (IL1B) to ∼ 110 b (CACNLG). All amplicons are detectable on the gel image. The analysis software assigned peak heights and scan numbers to each fragment detected above a default threshold value. This data was displayed as an electropherogram, where the relative fluorescence as a function of time of electrophoresis, represented by the scan number, is shown for all detected peaks. Each of the eight products from the eight individuals was assigned a genotype after analysis of the electropherograms. Representative electropherograms for each genotype are shown in Figure 5.

Peak height ratios for the relevant colors were determined for all electropherograms. The results are summarized for each locus. The IL1B genotypes determined by *Aval* enzyme digests were 6 CC homozygotes, 1 TT homozygote, and 1 CT heterozygote. The blue peak height (C allele) to green peak height (T allele) ratio for the CC homozygotes were 6.5 (panel A), 7.3, 10.1, 11.4, 9.3, and 1.8 (panel D). This last individual showed an elevated green peak that prevented a definitive genotype, either CC or CT, from being assigned. The green: blue peak height ratio was 6.7 for the TT homozygte (panel B) and was 1.1 for the CT heterozygote (panel C). The ALAD genotypes determined by *Rsa*I enzyme digestion identified 5 TT homozygotes, 2 CC homozygotes, and 1 CT heterozygote.

There were no green peaks detected in either CC homozygote, indicating they were not present above the default threshold setting of 20. Using this value, blue: green peak height ratios for the CC homozygotes were 22 (panel G) and 46. The DRD2a genotypes determined by *Hinc*II enzyme digestion identified 7 GG homozygotes and one GC heterozygote. The blue peak height (G allele) to yellow peak height (C allele) ratio for the GG homozygotes was 8 (panel H), 6, 89, 90, 152, 89, and 115. The individuals with a ratio above 10 had no detectable yellow peak so the default setting of 20 was used. This ratio was 3.0 for the CT heterozygote (panel I). The 5-HT2a genotypes determined by *Msp*I digests were 3 TT homozygotes, 3 CC homozygotes, and 2 CT heterozygotes. The green peak height (T allele) to blue peak height (C allele) ratio for the TThomozygotes was 13.8 (panel J), 5.0 and 4.5. The blue: green peak height ratio for the CC homozygotes was 10 (panel K), 3.0 and 2.0. The same ratio for the heterozygotes was 1.0 (panel L) and 0.7. The DCP1 genotypes determined by *BsmB*I restriction enzyme digests identified 6 CC homozygotes, 1 TT homozygote, and 1 CT heterozygote. The 6 CC homozygotes all showed a clear yellow peak (C allele) with no detectable red peak (T allele) signal. The yellow: red peak height ratio, using the default value of 20 for the red peak, was 113 (panel M), 170, 131, 120, 133, and 118. The TThomozygote showed a red: yellow peak height ratio of 12.6 (panel N). The same ratio for the single CT heterozygote was 1.3 (panel O). The DRD2 genotypes determined by *Taq*I restriction enzyme digests identified 4 TT homozygotes and 4 CT heterozygotes. The red peak height (T allele) to yellow (black) peak height (C allele) ratio for the TThomozygotes was 21 (panel P), 21, 18, and 39. This last individual had no detectable yellow background peak. The same ratio for the CT heterozygotes was 1.0 (panel Q), 0.8, 0.7, and 0.7. The DRD5 genotypes determined by *Eco*57I restriction enzyme digestion and DNA sequencing identified 8 CC homozygotes. The blue peak height (C allele) to green peak height (T allele) ratio was 6.1 (panel R), 3.2, 3.4, 3.5, 4.1, 4.2, 5.4 and 5.6. The genotypes for CACNLG determined by *Aci*I restriction enzyme digests were 7 GG homozygotes and 1 AA homozygote. All 7 GG homozygotes showed a clear yellow peak (G allele), with no background red peak (A allele) reaching the minimum detection threshold of 20 in any of the samples. The yellow: red peak height ratio for these individuals was 98 (panel S), 31, 125, 62, 102, 48, and 19. The single AAhomozygote had a red to yellow peak height ratio of 4.5 (panel T). This collective data set shows that 63 out of 64 genotypes determined by the multiplexed solid-phase amplification and minisequencing method were accurately determined.

The data provided above demonstrate a rapid and accurate method for determination of polymorphisms in nucleic acids samples. This system is capable of highly multiplexed PCR, as demonstrated with 54 beads, each with a unique primer-pair, in a single reaction chamber. This system combines the sensitivity of PCR and fluorescent detection with the capability of high multiplexing, creating an accurate, robust approach to polymorphism genotyping.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

## Claims

1. A method of determining the identity of a polymorphic nucleotide, said method comprising:
contacting under hybridizing conditions, a target nucleic acid comprising a polymorphic site, and a solid substrate comprising one or more bound locus-specific primer pairs;
amplifying said target nucleic acid with said locus-specific primer pair, wherein said amplifying results in an amplification product bound to the solid support at each end;
contacting said amplification product with a labeled probe comprising at least one detecting nucleotide that will specifically base pair with said polymorphic nucleotide, in the presence of an enzyme that catalyzes the formation of a covalent bond between said detecting nucleotide and said amplification product; and
detecting said label;
wherein the identity of the label on said detecting nucleotide indicates the complement of the polymorphic nucleotide.

2. The method according to Claim 1, wherein said enzyme is DNA polymerase.

3. The method of claim 2, wherein said amplification product is denatured and contacted with an extension primer that hybridizes to a site immediately adjacent to said polymorphic nucleotide,prior to contacting with said labeled probe, wherein DNA polymerase extends from said extension primer to covalently attach a labeled probe to its 3' end.

4. The method of Claim 2, wherein said amplification product is cleaved with an endonuclease to generate a free end; and
cleaving with a distance-cleaving endonuclease, resulting in a cleavage product having an overhang strand and a recessed strand comprising a 3' terminus, wherein the polymorphic nucleotide is on the single-stranded overhang of the cleavage product, wherein said recessed strand provides an extension primer for said DNA polymerase.

5. The method of claim 2, wherein said amplification product is contacted with a plurality of labeled probes selected from the group consisting of at least two differentially labeled dideoxynucleotides.

6. The method of claim 3, wherein a plurality of mutually distinguishable extension primers are used.

7. The method of Claim 1, wherein said enzyme is ligase.

8. The method of claim 7, wherein said amplification product is cleaved withan endonuclease to generate a free end; and
cleaving said amplification product with a distance-cleaving endonuclease, resulting in a cleavage product having a single-stranded overhang strand and a recessed strand, wherein the recessed strand has a 3' terminus, wherein the polymorphic nucleotide is on the single-stranded overhang of the cleavage product, wherein said recessed strand; contacting with ligase and at least one nucleotide complementary to said polymorphic nucleotide under conditions that permit covalent linkage.

9. The method of claim 8, wherein said amplification product is contacted with a plurality of differentially labeled oligonucleotide probes selected from the group consisting of all possible sequences of said single-stranded overhang.

10. The method of claim 10, wherein at least two different labels are used.

11. The method of claim 8, wherein the polymorphic nucleotide is the first nucleotide on the single-stranded overhang of the cleavage product.

12. The method of Claim 1, wherein said amplification product comprising said detecting nucleotide is released from said substrate for detection.

13. The method of Claim 1, wherein said amplification product comprising said detecting nucleotide is detected *in situ.*

14. The method according to Claim 1, wherein said solid substrate comprises a capture primer.
